(19) **Europäisches Patentamt / European Patent Office / Office européen des brevets**

(11) **EP 1 915 126 B1**

(12) **EUROPEAN PATENT SPECIFICATION**

(45) Date of publication and mention
of the grant of the patent:
**11.12.2013 Bulletin 2013/50**

(21) Application number: **06769661.7**

(22) Date of filing: **15.08.2006**

(51) Int Cl.:
*A61K 8/97* (2006.01)     *A61K 8/49* (2006.01)
*A61K 8/63* (2006.01)     *A61K 8/35* (2006.01)
*A61K 8/365* (2006.01)     *A61Q 19/08* (2006.01)

(86) International application number:
**PCT/SE2006/050282**

(87) International publication number:
**WO 2007/021240 (22.02.2007 Gazette 2007/08)**

(54) **Dermatologic composition**

Dermatologische Zusammensetzung

Composition dermatologique

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **18.08.2005 SE 0501834
11.01.2006 US 758449 P**

(43) Date of publication of application:
**30.04.2008 Bulletin 2008/18**

(73) Proprietor: **Tricutan AB
566 33 HABO (SE)**

(72) Inventors:
• **FREDRIKSSON, Lars
S-187 41 Täby (SE)**
• **STARLANDER, Ulf
S-113 35 Stockholm (SE)**

(74) Representative: **Ström & Gulliksson AB
Box 4188
203 13 Malmö (SE)**

(56) References cited:
**EP-A1- 1 108 419     WO-A2-2005/034891**

• **GROSSMAN R.: 'The role of
Dimethylaminoethanol in Cosmetic
Dermatology' AMERICAN JOURNAL OF
CLINICAL DERMATOLOGY vol. 6, no. 1, 2005,
pages 39 - 47, XP003004225**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field

The present invention relates to a new cosmetic composition for improving firmness and elasticity of the skin. In particular, the present composition comprises compounds exerting different modes of action in the skin.

### Background

Skin is a very complex organ and, by weight, the largest of the body build up of several layers - the epidermis, the dermis and the subcutaneous tissue. It provides the front line of defence for the body and is involved in processes of exchange between the internal and external environments.

Skin areas exposed to sun light or UV radiation show characteristic clinical changes such as hyper- and hypo pigmentation, wrinkles, pigmented lesions, benign lesions, keratoses, xerosis, roughness etc. Areas most affected, such as hands and face, are also those most visible in our social life.

The most common cell in dermis is the fibroblast. The cell produces fibre "threads" such as collagen, elastin and reticulin. The fibres are imbedded in the watery basic substance. The water binding substances in the basic substance are glycosaminoglycans that are also produced by the fibroblasts.

The fibroblasts also produce collagenase or metalloproteinases (MMPs). MMPs form a certain group of enzymes that split proteins in the dermis and the basal membranes. Paradoxically, fibroblasts also produce MMP-inhibitors (TIMPs). To keep dermis unaffected there has to be a balance between the two opposite activities.

Ageing decreases collagen synthesis in dermis. Environmental stress such as smoking, UV radiation and inflammations stimulate MMPs and accelerate collagen degradation. These activities disturb the balance in dermis. The activity and production of MMPs and TIMPs are regulated by proteins that are formed in skin cells, so called cytokines. Anti-inflammatory substances can inhibit MMP activation and thus stop collaged breakdown. Another method to stop collagen degradation in dermis is direct inhibition of MMPs as such.

Ageing and photo damages cause neutrophiles to penetrate dermis, thereby releasing elastase. When the balance is disturbed, elastase will break down elastin and at the same time activate processes resulting in inflammation. The neutrophiles attract macrophages. Macrophages in turn release inflammatory cytokines such as TNF, interleukin-1 and -8 that stimulate even more neutrophiles. The cytokines cause a chain of reactions that increase the release of elastase. Activation of lipooxygenase and cyclooxygenase increases the production and  release of inflammatory mediators such as prostaglandins and leukotriens. All of the above events result in MMP synthesis and/or stimulation of enzymatic activity of the MMPs.

Skin appearance is markedly influenced by the condition of structural proteins in dermis as well as glycosaminoglycans, foremost hyarulonic acid. In childhood, fibroblast synthesis exceeds the enzymatic activity, which result in a youthful appearance. With ageing and UV radiation, the relationship is the opposite, which in time will render the skin an aged appearance. Stimulating processes that increase the synthesis of the structural proteins and glycosaminoglycans and/or inhibiting processes that lead to increased production of enzymes such as collagenase, elastase and hyaluronidase, it could be possible to influence the balance between synthesis and break down.

Anti-wrinkle products have been on the market for some time, few of them having effect on wrinkles. However, to achieve a youthful appearance, it is much more important to treat slack and sagging facial skin. Young people may have wrinkles, but only elderly have sagging skin.

It would be desirable to obtain a composition based on well known constituents, which counteracts or ameliorates the aforementioned conditions involved in ageing and the conditions resulting from exposure to UV radiation, negatively affecting the condition and appearance of the skin.

Large numbers of active substances influencing the condition of the skin are known. For example, dimethylaminoetanol (DMAE) is known to improve skin firmness. It has been proposed that the activity of DMAE is related to DMAE acting as a precursor to acetylcholine, which may explain the increased levels of neurotransmitter providing increased tone for the muscles. Muscle tone under the skin improves the appearance of sagging tissue. The role of DMAE in cosmetic dermatology is reviewed by Grossman in Am J Clin Dermatol 2005:6(1):39-47. Grossman presents indications of a possible double action of DMAE. A muscle contracting action which does not happen earlier than seven hours after application and therefore cannot explain the short-term action which usually arises within 15 minutes after application. This short-term action may be explained by acetylcholine working in the epidermis as a local hormone with autocrine and paracrine functions making cells in epidermis coming closer to each other making it possible to increase skin firmness.

Asiatic acid stimulates the fibroblast synthesis of collagen (type I and III) in dermis. Some studies have also shown a regulating function of abnormal connective tissue growth by extract from *Centella asiatica* comprising asiatic acid. Asiatic acid can regenerate collagen of connective tissue in bad condition, both in quantity and quality.

Extract from *Centella asiatica* has been used locally with positive results for vein problems (including a double-blind study), striae gravidarum (double-blind, prophylactic study) and cosmetic cellulite (open study).

Ursolic acid stimulates collagen production in dermis and increases cell generation in the basal layer. Ursolic acid inhibits elastase as well as the release of inflammatory cytokines, such as lipooxygenas and cyclooxygenase. Both ursolic acid and asiatic acid inhibit the production of UVA induced reactive oxygen, lipidperoxidation and induction of MMP-2 which indicates the possibility that the substances can be valuable for treatment of photo damaged skin.

WO 2005/034891 relates to an anti-ageing composition comprising at least one plant ingredient or plant extract from *Rosmarinus officinalis*, at least one plant ingredient or plant extract from *Centella*, *Echinacea* or *Alpinia* and at least one DNA repair enzyme. The composition is evaluated by measuring the moisture content of the skin but otherwise only visually, making it difficult to scientifically evaluate and compare the composition of WO 2005/034891 with other anti-ageing compositions. Further, WO2005/03489 does not indicate improvement of sagging skin/firmness just improvement of moisturization.

Curcumin is another known substance affecting the skin. Animal tests show that curcumin accelerates wound healing and influences skin growth factors. Furthermore, curcumin inhibits formation of collagenase, elastase, and hyaluronidase and inhibits, similar to ursolic acid, lipoxygenas and cyklooxygenas. Curcumin also suppresses the gene expression of the transcript factors c-jun and c-fos, which must be combined for formation of activator protein-1 (AP-1). AP-1 can activate the MMP genes, which results in formation of collagenase, elastase and stromelysin that have been shown to fully break down skin collagen. Curcumin induces formation of heme-oxygenas (HO-1). HO-1 has a key function for re-establishing cell balance and could be of importance in inflammatory processes in the skin.

Curcumin obstructs irreversible cross-bindings between glucose and collagen, which are developed of AGE (Advanced Glycosylation End Products). Cross bindings contribute to stiffness and lack of elasticity in skin tissue and to the thickening of capillaries that can be observed with diabetes and ageing. Curcumin also inhibits tyrosinase, which makes it possible to reduce age related pigment spots. Curcumin has also immune modulating characteristics.

Animal tests show a fast transformation of curcumin to dihydrocurcumin and tetrahydrocurcumin (THC or tetrahydrodiferuloylmethane). It has been shown that THC is the dominant metabolite *in vivo.* THC retains the mechanisms of curcumin in important aspects; thus, a low serum concentration of curcumin does not necessarily mirror the total biological effect of curcumin. Curcumin is used as colorant in food and tends to render the skin a yellow colour. Hence, THC has been used in cosmetic products, as it is neutral in colour.

EP 1 108 419 describes a cosmetic composition comprising an essential antioxidant for protection of keratinous tissue against environmental stress, such as smoke, smog and UV radiation. The antioxidant in the composition is hesperetin, tetrahydrocurcumin, tetrahydromethoxycurcumin or tetrahydrobisdemethoxycurcumin or mixtures thereof. EP 1 108 419 discloses protection of keratinous tissue against environmental stress. This might explain how to protect or diminish sagging of the skin in the future but does not explain how this might influence skin which already is sagging.

In order to establish a composition which counteracts or ameliorates the effects of ageing and/or UV damaged skin, the present inventors suggest a novel selection of constituents, each with a documented, different bioactivity and mode of action.

**Summary of the invention**

The present invention relates to a unique composition of active substances according to claim 1 for improving firmness and elasticity of the skin. The composition comprises asiatic acid, tetrahydrodiferuloylmethane, ursolic acid and dimethylmonoaminoethanol (DMAE).

**Description of the invention**

One object of the present invention is to provide a cosmetic composition for increasing the firmness and elasticity of the skin, thus preventing or decreasing the anti-ageing process of the skin and in particular of photo-damaged skin.

Another object is to provide a cosmetic composition increasing the firmness and elasticity of the skin, making the skin look younger.

A further object of the present invention is to provide a cosmetic composition with capacity to affect the facial skin in three ways - i.e. the epidermis, the dermis (cutis) and the muscular layer underneath the subcutaneous layer, whereby the constituents of the composition are purposefully selected so that they simultaneously exert their respective activity at predetermined locations without such interactions that reduce or abolish their expected activities.

The composition of the present invention according claim 1 comprises a unique combination of asiatic acid, tetrahydrodiferuloylmethane (tetrahydrocurcumin, THC) and ursolic acid with efficacy to improve firmness and elasticity of the skin. The composition further comprises DMAE (dimethylmonoaminoethanol) in order to achieve a particularly deep-going effect involving the facial muscles underlying the skin.

As used herein, an increase/improvement of the firmness and elasticity of the skin is defined as a change of the facial skin determined by measuring skin sagging as outlined, below in Example 2 in the detailed description. The size of the increase/improvement of the firmness and elasticity is determined by a reduction of a sagging quotient (SQ) measured before the start of the treatment and after the treatment. An increase/improvement of the firmness and elasticity of the skin is also defined as a change of the facial skin determined by measuring Resonance Running Time (RRTM) as outlined in Example 4 in the present description. The size of the increase/improvement of the firmness and elasticity is determined by a reduction of a RRTM value measured before the start of the treatment and after the treatment. Preferably the RRTM value decreases at least 5 %, preferably at least 10% and more preferably at least 20%.

In the inventive compositions, the concentration of asiatic acid is 0.08-2 percent by weight and more preferably 0.1 % by weight, while the concentration of tetrahydrodiferuloylmethane in the inventive composition is 0.1-1 % by weight and more preferably 0.1 % by weight, and the concentration of ursolic acid is 0.08-2 % by weight and more preferably 0.1 % by weight.

DMAE is present in amount of 2-4 % by weight and more preferably about 3 % by weight.

The composition comprises asiatic acid at a concentration of about 0.08-2 % by weight, tetrahydrodiferuloylmethane at a concentration of about 0.1-1 % by weight and ursolic acid in a concentration of about 0.08-2% by weight, and DMAE in a concentration of about 2-4 % by weight.

In yet another embodiment of the present invention the composition comprises asiatic acid at a concentration of about 0.1 % by weight, tetrahydrodiferuloylmethane in a concentration of about 0.1 % by weight and ursolic acid in a concentration of about 0.1 % by weight, and DMAE at a concentration of about 3 % by weight.

The asiatic acid can be extracted from natural botanical sources, such as plants, but can also be produced synthetically. In the present invention the asiatic acid is present in an extract from *Centella asiatica.* Preferably the concentration of asiatic acid in the *Centella asiatica* extract is at least 50 % and more preferably at least 50-95%.

The ursolic acid can be extracted from natural botanical sources, such as plants, but can also be produced synthetically. In the present invention the ursolic acid is present in an extract from *Rosmarinus officinalis* leaves. Preferably the concentration of ursolic acid in the *Rosmarinus officinalis* leaf extract is at least 50 % and more preferably at least 50-95%.

Examples of other botanical sources of ursolic acid are, but not limited to, *Ligustrum japonicum, Plantago asiatica, Plantago major, Ocimum basilicum,* and *Prunus* species. Alternatively, the ursolic acid is comprised in liposomes. Liposomic ursolic acid increases ceramid production in human skin. Amphiphilic substances with capacity of forming liposomes with conventional methods are well known for a person skilled in the art. For example, lecithin can be used as an amphiphilic substance for liposome formation.

The composition of the present invention is preferably applied in combination with a cosmetically acceptable carrier suitable for topical administration. The cosmetically acceptable carrier is capable of having the active substances and any other ingredients of the composition dissolved therein, and possesses acceptable safety properties. Cosmetically acceptable carriers suitable for topical administration are well known to a person skilled in the art. Examples of cosmetically acceptable carriers suitable for topical administration are, but not limited to, conventional gel bases, cream bases, serum bases and oil bases.

The inventive composition may be in the form of, for example but not limited to, gels, lotions, ointments, creams, oils, sprays, sticks or any other form suitable for topic administration.

The present invention also relates to a non-therapeutic anti-ageing cosmetic method for improving the firmness and elasticity of the skin. In the method a composition as described above is applied to the skin, preferably at least once a day and preferably at least twice a day.

To the best knowledge of the present inventors, this is the first time a combination of asiatic acid, tetrahydrodiferuloylmethane (tetrahydrocurcumin,) and ursolic acid, in combination with DMAE, is used as a cosmetic anti-ageing product, and in particular to increase firmness and elasticity of photo-damaged skin. Each one of the active substances has been carefully selected in order to obtain a composition that after application to the skin gives a genuine "lift". As shown by the examples below this "lift" has been evaluated, not only visually, but also scientifically by measurement by two different well known methods.

**Detailed and exemplifying part of the description**

**Brief description of the drawings**

Figure 1 is a diagram illustrating the reduction of skin sagging after long-term application with a composition comprising a cream base and *Centella asiatica* extract, *Rosmarinus Officinalis* leaf extract and tetrahydrodiferuloylmethane.

Figure 2 is a diagram illustrating the reduction of UV-spots after long-term application with a composition comprising a cream base and *Centella asiatica* extract, *Rosmarinus Officinalis* leaf extract and tetrahydrodiferuloylmethane.

Figure 3 is a diagram illustrating the reduction of skin sagging after short-term treatment with a composition comprising a serum base and *Centella asiatica* extract, *Rosmarinus Officinalis* leaf extract, tetrahydrodiferuloylmethane and DMAE.

Figure 4 is a diagram illustrating the increase in skin firmness measured using a reviscometer.

Without being bound to particular theories, the present inventors contemplate that by stimulating processes that increase the synthesis of the structural proteins and/or inhibiting processes that lead to degradation of the skin it could be possible to influence the balance between synthesis and degradation. Asiatic acid, ursolic acid and THC inhibit enzymatic degradation and destructive inflammatory processes in skin. At the same time they increase skin growth factors and collagen synthesis. While asiatic acid, ursolic acid and THC improve skin tissue elasticity and firmness by influencing structural proteins and water-binding substances, DMAE influences facial muscle contraction and keratinocyte compactness. The former processes have a long-term effect on skin firmness whilst the effect of DMAE can be noticed more or less instantly. Thus, the composition according to the present invention affect the skin through:

- a short-term action making cells in epidermis to come closer to each other,
- a middle-term action by contracting facial muscles upon which skin layers lie making the bedding of muscles smoother,
- a long-term action increasing the synthesis of structural proteins and glycosaminoglycans in dermis which result in less sagging of skin.

Skin firmness is influenced by facial muscle contraction. There is evidence that enhanced endogenous levels of acetylcholine in facial muscle synapses after topical application of DMAE could improve their capacity to contract. The layer of facial muscles supporting the skin would in that case become firmer as well as the skin covering the muscles. Such a mechanism could possibly be valid also for long-term changes. More or less instant noticeable skin alterations after application of DMAE is however supposed to be due to non-neuronal signal networking systems present in epidermis. It has been shown that such systems exist and that keratinocytes, the dominating cell in epidermis, emit and have receptors for acetylcholine. Increased levels of acetylcholine are supposed to have an effect on compactness of keratinocytes and consequently skin firmness.

However, DMAE and substances stimulating processes that increase the synthesis of the structural proteins and glycosaminoglycans and/or inhibiting processes that lead to increased production of enzymes such as collagenase, elastase and hyaluronidase result in less slagging of skin than DMAE alone after one months' use.

The composition according to the present invention comprises complex substances that can act together to treat the epidermis, the dermis and the subcutaneous tissue. Each one of asiatic acid, ursolic acid and THC can exert its mode of action in the skin in the presence of each other. The following experimental data indicate that the three compounds simultaneously retain their biological effects without affecting the efficiency of one another. It is not unusual that biologically active compounds produce reactive metabolites that can affect other active compounds occupying the same location or that the biologically active compound itself interact with other active compounds.

It is evident that the selected compounds in the composition of the following examples affect the epidermis, the dermis (cutis) and the subcutaneous tissue, as well as the layer of facial muscles that underlies the skin, simultaneously maintain their biological effect in the complex biosystem of the skin.

The present invention now will be further described in the following detailed description including non-limiting examples.

**Examples**

**Example 1**

Table of contents of one illustrative composition according to the present invention.

TABLE OF CONTENTS

| | Percent by weight |
|---|---|
| Aqua | 81.48 % |
| Lactic Acid | 4.0 % |

(continued)

| | Percent by weight |
|---|---|
| Dimethylmonoaminoethanol (DMAE) | 3.0 % |
| Propylene Glycol | 3.0 % |
| Ammonium Acryloyldimethyltaurate/VP Copolymer | 2.2 % |
| Polyglycerin-6 | 2.0 % |
| PEG-16 Macadamia Glycerides Butylene Glycol (and) | 2.0 % |
| Centella Asiatica Extract | 1.0 % |
| Phenoxyethanol (and) Ethylhexylglycerin | 0.7 % |
| Rosmarinus Officinalis Leaf Extract (and) Lecithin | 0.3 % |
| (Perfume* | 0.22 %) |
| Tetrahydrodiferuloylmethane | 0.1 % |

\* If perfume is added to the composition the water content decreases correspondingly. Perfume is usually present at a concentration of about 0.2 % as in example 1.

**Example 2. Double blind split-face study using a composition comprising *Centella asiatica* extract, *Rosmarinus Officinalis* leaf extract and tetrahydrodiferuloylmethane**

In this example a long-term study was performed using a composition comprising as active substances *Centella asiatica* extract, *Rosmarinus Officinalis* leaf extract and tetrahydrodiferuloylmethane in a cream base.

Study Design

Participants: 33 healthy persons, 35 to 74 years.
Duration: 84 days.
Application: The study was performed as a double blind split face study. The participants received two compositions in separate flasks, one comprising only the cream base (placebo) and the other comprising the cream base and the active substances: Twice daily, a certain volume of one of the compositions was applied to the left hand side of the face and the same volume of the other composition was applied to the right hand side of the face.
Measuring of skin sagging (described below): SQ-values were measured both on the left hand side and the right hand side of the face before the start of the treatment and after 84 days of treatment.
Pigmentation: The number of UV spots was measured both on the left hand side and the right hand side of the face visually before the start of the treatment and after 84 days of treatment using a digital photo technique.

Measuring skin sagging

In this study a method to assess changes in skin properties related to firmness by measuring the sagging or deformation of facial skin due to different forces, but mainly the force of gravity which is responsible for sagging observed under real life conditions, was used. In this method, subjects are first placed in a reclining position, with their face directed towards the ceiling. One or more marks are placed on the cheek and lateral chin area, and the distance is precisely measured, using a modified Vernier calliper, from a fixed feature on the face such as the centre of the eye when focused directly forward. Subjects are then either inclined into a normal upright position, or further declinated so their body axis is -45° in orientation with respect to a horizontal body axis. The change in distance from the fixed reference point is re-measured.
From these measurements, a sagging quotient (SQ) for each point marked on the face is determined as follows:

$$SQ = (\Delta d1)^2 + (\Delta d2)^2$$

Where d1 is the distance of the marked point measured at rest and then re-measured when subject is upright, and d2 is the distance from the marked point measured at rest and then re-measured in the declinated position.
To help visualize, the marked point is usually defined to the centre of the cheek. The distance is measured to the centre of the eye in the reclined position, a position in which gravity is not pulling the mark away or towards the eye. When the subject is re-positioned upright, the marked point moves away from the eye. The more the skin sags the greater the point moves. The increase in distance the point moves is defined as the change in d1 (distance at rest minus distance inclined, i.e. $\Delta d1$).

Next the subject is declinated, which means the head is placed lower than the rest of the body. In this case the marked point will move towards the eye. The poorer the skin condition, the more the skin sags and the closer the point moves towards the eye. The distance to the eye is defined as d2. The change in d2 is the distance at rest minus distance inclined. Greater changes in d1 and d2 upon change in subject inclination result in greater changes in SQ.

The SQ values obtained before treatment are compared with those obtained after treatment to determine the significance of product effects. Using this analysis, data on facial skin from various age groups of subjects show dramatic increases in the SQ values with corresponding increases in age.

Results

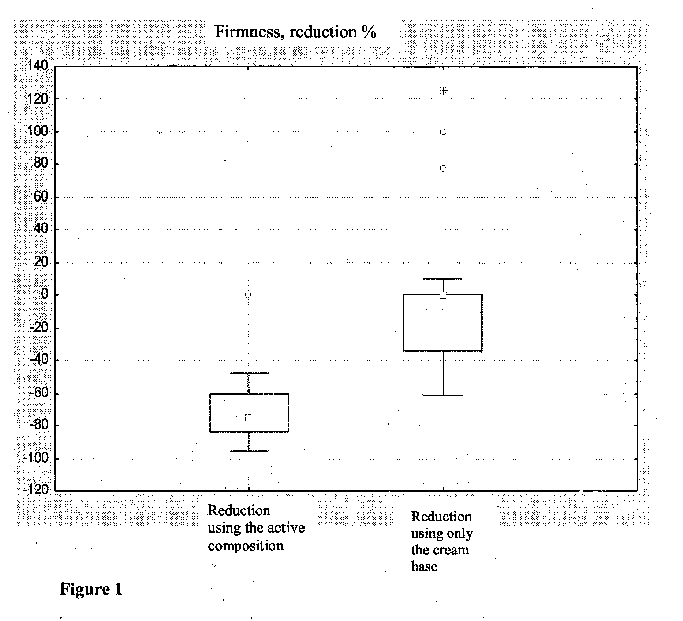

The results of the skin sagging measurements are shown in Figure 1 and were evaluated through Wilcoxon Matched Pairs Test. P-levels of <0.05 were significant.

Figure 1 shows a significant difference (p< .001) between the reduction of the SQ values using only the cream base compared to using the cream base comprising the active substances *Centella asiatica* extract, *Rosmarinus Officinalis* leaf extract and tetrahydrodiferuloylmethane.

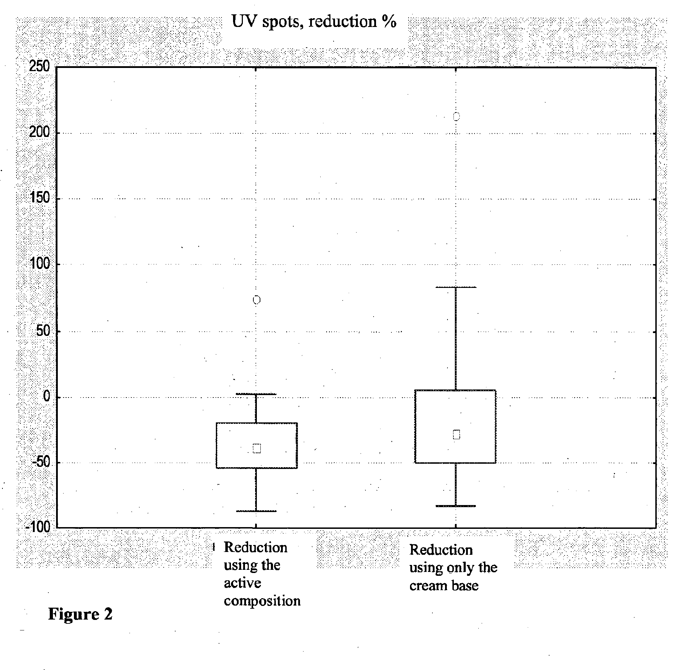

This study also showed a significant reduction of UV-spots (p=0.05) using the cream comprising the active substances (Figure 2). The results were evaluated through Wilcoxon Matched Pairs Test. P-levels of <0.05 were significant.

**Example 3: Double blind split-face study using a composition comprising *Centella asiatica* extract, *Rosmarinus Officinalis* leaf extract, tetrahydrodiferuloylmethane and DMAE**

In this example a short-term study was performed using a composition comprising as active substances *Centella asiatica* extract, *Rosmarinus Officinalis* leaf extract, tetrahydrodiferuloylmethane and DMAE in a serum base in order to evaluate the quick-action of the composition.

Study Design

Participants: 19 healthy persons.

Duration: 45 minutes.

Application: The study was performed as a double blind split face study. The participants received two compositions in separate flasks, one comprising only a serum base (placebo) and the other comprising the serum base and the active substances. A certain volume of one of the compositions was applied to the left hand side of the face and the same volume of the other composition was applied to the right hand side of the face.

Measuring of skin sagging (described in Example 2): SQ-values were measured both on the left hand side and the right hand side of the face before the start of the treatment and after 45 minutes.

Results

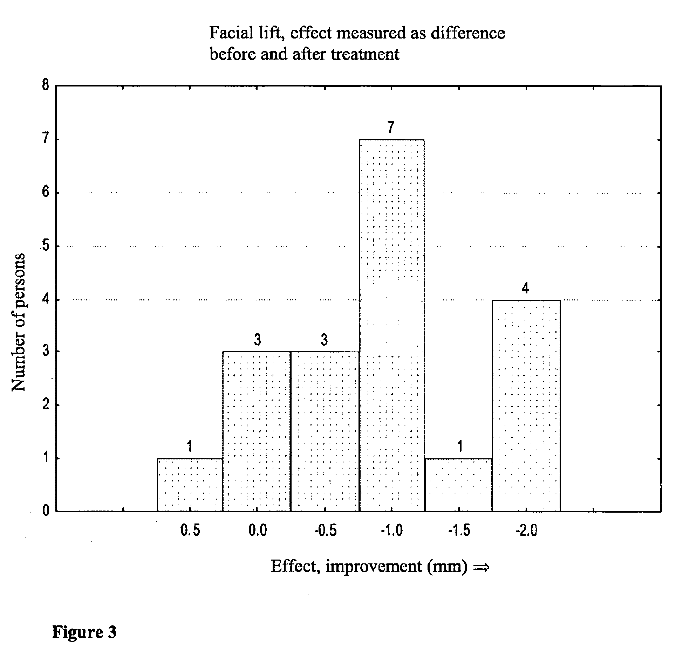

The results are shown in Figure 3 and were evaluated through a T-test for dependent samples. Marked differences were significant at p<0.05.

Fifteen of the participants showed a reduction in skin sagging, three persons showed no effect and one person showed a slight increase in skin sagging. The reduction of skin sagging was statistically significant (p<0.001).

**Example 4: Randomized, vehicle-controlled, double blind, split-face study on the clinical efficacy of a serum containing Centella asiatica extract, Rosmarinus Officinalis leaf extract, tetrahydrodiferuloylmethane and DMAE on sagging skin**

Increased consumer interest of surgical facial lifting has increased the demand for non-surgical solutions as well. The purpose of this study was to measure the clinical effect of a serum containing *Centella asiatica* extract, *Rosmarinus Officinalis* leaf extract, tetrahydrodiferuloylmethane and DMAE on sagging facial skin and whether it could serve as a preventive treatment or an alternative to a traditional face-lift.

Materials and methods

28 females (age 34-67) were included in the study. In order to standardize conditions during the test period subjects were instructed to use the same soap (Lactacyd®, GlaxoSmithKline). The test subjects were also supplied with a moisturizing cream containing canola oil and shea butter. Regarding make-up, the test subjects were advised to continue their daily routines, with the exception being on days scheduled for test controls, on which cosmetics was restricted.

*Test procedure*

The test procedure was done according to good clinical practice (GCP) guidelines.

The study consisted of three parts. A self-assessment form was filled in by the test subjects on two occasions, and was handed over to the study nurse before the clinical evaluation. Two clinical evaluations were made by the same dermatologist at the start of the study, and after four weeks of treatment. A clinical protocol divided into left and right side of the face was filled in at each visit. Estimate of the treatment effect was graded from 0 to 6 with 0 = worsening effect, 2 = no change and 6 = excellent result.

Skin firmness was measured using a Reviscometer (C+K Electronics, Cologne, Germany) recording Resonance Running Time (RRTM). Skin firmness is measured by assessing the speed of transmission of a 500-Mhz mechanical signal through the skin. A lower RRTM value is interpreted as firmer skin. The RRTM measurements were recorded at four occasions, at the start of the study (T0) before topical application of treatment gel and 15 minutes after application of a pea-size of gel (TO+15). The same procedure was repeated after 4 weeks with measurements at T1 and T1+15 (i.e. before and after application of gel). Measurements were taken on both cheeks at a fixed position of the probe. RRTM values were recorded in four directions (0°, 90°, 180°, 270°) with the average score being used as assessment of general skin firmness.

*Treatment gel*

Gels contained identical vehicle. The active gel also contained titrated extracts of *Centella asiatica* (asiatic acid, madecassic acid, asiaticoside), *Rosmarinus officinalis* (ursolic acid), tetrahydrocurcumin and dimethylaminoethanol (DMAE) but was otherwise identical in colour, smell and consistency to the control gel. Two coded 30 g bottles, marked right and left, respectively, for use twice daily on each half of the face during the 4-week treatment period, were distributed at the start and the first visit. A coded list was used to determine if the right or left side should use active product or vehicle.

*Statistical method and considerations*

The power analysis was based on an estimate of the proportion of subjects showing improvement on the active side. Using a $x^2$-test for proportions in a group of 28 subjects a power of 80% is achieved to detect the difference between the null hypothesis $P = 0\cdot5$ (active side preferred) and a hypothetical improvement $P = 0\cdot75$ for active side. A statistically significant difference in efficacy is achieved if the difference from the null hypothesis (no difference between active and control side) reaches $P < 0\cdot05$. A single tailed $P < 0\cdot1$ indicates a trend, but is not conclusive. Firmness was compared before and after 4 weeks of treatment. The difference between the active and the vehicle side was statistically analysed using the Wilcoxon matched pairs rank test (WT).

*Ethical considerations*

Subjects included in the study were recruited through an advertisement in a local newspaper or at dermatologists' receptions. They were informed about the study in writing and verbally. Subjects who met the inclusion criteria signed a written consent form before being allowed to enter the study. The study was approved by the Regional Ethical Review Board in Stockholm at Karolinska Institutet (EPN).

Results

Of those recruited, 25 of 28 subjects completed the 4-week treatment period according to the clinical protocol. The reason for discontinuing the study was a mild irritative contact dermatitis. However, other subjects had milder, temporary irritative reactions, manifested after 3-4 days, which then subsided and disappeared. This could imply that those discontinuing the study might have tolerated the product with less frequent applications. The results of the remaining 25 subjects were analyzed.

*Self assessment*

The self assessment showed significant improvement on the facial side treated with active cream (p<0.02). The result is presented in the figure below.

As shown in the figure, 14 subjects out of 22 (three did not fill in the self assessment form) rated the facial side treated with active cream as improved, of them 7 with excellent result. 8 subjects noted no difference on facial side treated with active gel. Six subjects reported improvement on control side whereas the majority, 16 subjects, noted no difference on control side.

*Clinical assessment*

The clinical assessment showed significant improvement on the facial side treated with active gel ($p<0.01$). Improvement on facial side treated with active gel was recorded on 19 subjects and no difference on 6 subjects. On control side, no difference was noted on 15 subjects, 5 subjects had worse results and 5 improved results.

*Result of Reviscometer measurementt*

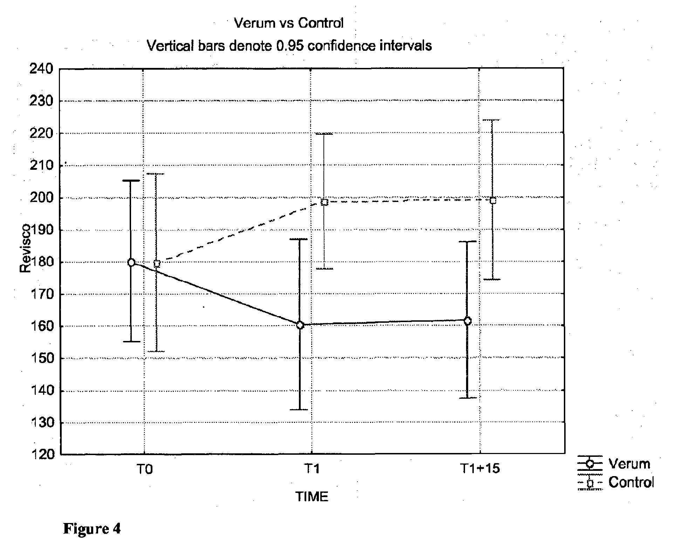

The Reviscometer measurements (using average score in four directions as presented above) showed significant improvement with $p<0,037$ between T0 and T1. No significant difference was recorded between T0 and T0+15 as well as T1 and T1+15. The result is presented in figure 4.

Conclusions

The study showed that the active gel with titrated extracts of *Centella asiatica*, *Rosmarinus officinalis*, tetrahydrocurcumin and DMAE had a significant firming effect on facial skin after four weeks of treatment. Objective measurements with a Reviscometer confirmed the dermatologist's clinical assessment as well as the improved firming effect experienced by the subjects. Whether topical treatments could be a realistic alternative to facial surgery must be further evaluated. However, the results suggest that topical treatments can be used for moderate improvement of sagging facial skin.

**Claims**

1. A composition for improving firmness and elasticity of the skin,
   wherein the composition comprises

   a. *Centella asiatica* comprising asiatic acid, wherein asiatic acid is present in an amount of 0.08-2 % by weight of the composition,
   b. *Rosmarinus officinalis* comprising ursolic acid, wherein ursolic acid is present in an amount of 0.08-2 % by weight of the composition,
   c. 0.1-1 % by weight tetrahydrodiferuloylmethane and
   d. 2-4 % by weight dimethylmonoaminoethanol (DMAE)

2. A composition according to claim 1, wherein the concentration of DMAE is 3 % by weight.

3. A composition according to claims 1, wherein the concentration of Asiatic acid is 0,1 % by weight.

4. A composition according to claims 1, wherein the concentration of tetrahydrodiferuloylmethane is 0.1 % by weight.

5. A composition according to claim 1, wherein the concentration of ursolic acid is 0.1 % by weight.

6. A composition according to claim 1, wherein the concentration of DMAE is 3 % by weight, Asiatic acid is 0,1 % by weight,
   tetrahydrodiferuloylmethane is 0.1 % by weight and ursolic acid is 0.1 % by weight.

7. A composition according to claim 1, wherein the concentration of DMAE is 3 % by weight, *Centella asiatica* is 1,0 % by weight, tetrahydrodiferuloylmethane is 0.1 % by weight and *Rosmarinus officinalis* is 0.3 % by weight.

8. A non-therapeutic method for improving the firmness and elasticity of the skin, wherein a composition according to any one of the claims 1-7 is applied to the skin.

**Patentansprüche**

1. Zusammensetzung zur Verbesserung von Festigkeit und Elastizität der Haut, wobei die Zusammensetzung umfasst:

   a. *Centella asiatica* umfassend Asiatsäure, wobei Asiatsäure in einer Menge von 0,08-2 Gew.-% der Zusammensetzung vorhanden ist,
   b. *Rosmarinus officinalis* umfassend Ursolsäure, wobei Ursolsäure in einer Menge von 0,08-2 Gew.-% der Zusammensetzung vorhanden ist,
   c. 0,1-1 Gew.-% Tetrahydrodiferuloylmethan und
   d. 2-4 Gew.-% Dimethylmonoaminoethanol (DMAE).

2. Zusammensetzung nach Anspruch 1, wobei die Konzentration von DMAE 3 Ges.-% beträgt.

3. Zusammensetzung nach Anspruch 1, wobei die Konzentration von Asiatsäure 0,1 Gew.-% beträgt.

4. Zusammensetzung nach Anspruch 1, wobei die Konzentration von Tetrahydrodiferuloylmethan 0,1 Gew.-% beträgt.

5. Zusammensetzung nach Anspruch 1, wobei die Konzentration von Ursolsäure 0,1 Gew.-% beträgt.

6. Zusammensetzung nach Anspruch 1, wobei die Konzentration von DMAE 3 Ges.-% beträgt, von Asiatsäure 0,1 Gew.-% beträgt, von Tetrahydrodiferuloylmethan 0,1 Gew.-% beträgt und von Ursolsäure 0,1 Gew.-% beträgt.

7. Zusammensetzung nach Anspruch 1, wobei die Konzentration von DMAE 3 Ges.-% beträgt, von *Centella asiatica* 1,0 Gew.-% beträgt, von Tetrahydrodiferuloylmethan 0,1 Gew.-% beträgt und von *Rosmarinus officinalis* 0,3 Gew.-% beträgt.

8. Nicht-therapeutische Methode zur Verbesserung der Festigkeit und Elastizität der Haut, wobei eine Zusammensetzung nach einem der Ansprüche 1-7 auf die Haut aufgetragen wird.

**Revendications**

1. Composition pour améliorer la fermeté et l'élasticité de la peau, dans laquelle la composition comprend

   a. *Centella asiatica* comprenant de l'acide asiatique, dans lequel l'acide asiatique est présent dans une quantité allant de 0,08 à 2 % en poids de la composition,
   b. *Rosmarinus officinalis* comprenant de l'acide ursolique, dans lequel l'acide ursolique est présent dans une quantité allant de 0,08 à 2 % en poids de la composition,
   c. 0,1 à 1 % en poids de tétrahydrodiféruloylméthane et
   d. 2 à 4 % en poids de diméthylmonoaminoéthanol (DMAE)

2. Composition selon la revendication 1, dans laquelle la concentration de DMAE est de 3 % en poids.

3. Composition selon la revendication 1, dans laquelle la concentration de l'acide asiatique est de 0,1 % en poids.

4. Composition selon la revendication 1, dans laquelle la concentration du tétrahydrodiféruloylméthane est de 0,1 % en poids.

5. Composition selon la revendication 1, dans laquelle la concentration de l'acide ursolique est de 0,1 % en poids.

6. Composition selon la revendication 1, dans laquelle la concentration du DMAE est de 3 % en poids, de l'acide asiatique est de 0,1 % en poids, du tétrahydrodiféruloylméthane est de 0,1% en poids et de l'acide ursolique est de 0,1 % en poids.

7. Composition selon la revendication 1, dans laquelle la concentration de DMAE est de 3 % en poids, de *Centella asiatica* est de 1,0 % en poids, de tétrahydrodiféruloylméthane est de 0,1 % en poids et de *Rosmarinus officinalis* est de 0,3 % en poids.

8. Procédé non thérapeutique pour améliorer la fermeté et l'élasticité de la peau, dans lequel une composition selon l'une quelconque des revendications 1 à 7 est appliquée sur la peau.

**Figure 1**

**Figure 2**

Facial lift, effect measured as difference
before and after treatment

**Figure 3**

Verum vs Control
Vertical bars denote 0.95 confidence intervals

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005034891 A
- WO 200503489 A

- EP 1108419 A

**Non-patent literature cited in the description**

- **GROSSMAN.** *Am J Clin Dermatol,* 2005, vol. 6 (1), 39-47